(19) Europäisches Patentamt
European Patent Office
Office européen des brevets

(11) **EP 4 006 911 A1**

(12) **EUROPEAN PATENT APPLICATION**

(43) Date of publication:
**01.06.2022 Bulletin 2022/22**

(21) Application number: **21211049.8**

(22) Date of filing: **29.11.2021**

(51) International Patent Classification (IPC):
***G16H 10/60*** *(2018.01)*

(52) Cooperative Patent Classification (CPC):
**G16H 10/60**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **27.11.2020 IT 202000028760**

(71) Applicant: **Piqubo S.p.A.**
**40038 Vergato (BO) (IT)**

(72) Inventor: **PALMIERI, Tommaso**
**40046 Frazione di Porretta Terme, Alto Reno
Terme
(Bologna) (IT)**

(74) Representative: **Petruzziello, Aldo**
**Racheli S.r.l.**
**Viale San Michele del Carso, 4**
**20144 Milano (IT)**

(54) **METHOD FOR TRACKING CONTACTS**

(57) A description is given of a method for tracing contacts occurring among a group of users (U) within a space in which a plurality of detection stations (S) are set up, wherein:
- having selected a reference user (U$_{ref}$) from among the group of users (U), identifying for each station (Sj) all the passages carried out (t$_{j,ref}$) by one or more tracing means (M$_{ref}$) assigned to said reference user (U$_{ref}$);
- for each station (Sj), having established an interval of probably contact (Δt$_j$), among all the passages (t$_{j,i}$) de-

termining possible passages of users having come into contact (tj,contact) such that the following condition occurred:

$$t_{j,ref} - \Delta t_j < t_{j,contact} < t_{j,ref} + \Delta t_j$$

and from the passages determined identifying the identity of the users who came into contact with the reference user (U$_{ref}$).

FIG.1

Processed by Luminess, 75001 PARIS (FR)

EP 4 006 911 A1

**Description**

The present invention relates to a method for tracing contacts that have occurred between a group of users. More particularly, the present invention describes a method for tracing any contacts that a reference person has had with other people within circumscribed spaces, such as ski resorts, amusement parks, trade fairs and exhibitions, railway stations, public transport, and so on.

Background of the invention

Contact tracing systems and methods have taken on a fundamental quality above all following the recent pandemic due to the spread in 2020 of the new coronavirus commonly referred to as COVID-19. The spread of this virus has involved practically all countries on all continents, causing profound disruptions in the lives of the world's population.

Various measures have been adopted to limit or circumscribe the spread of the virus. The most significant among these consists of the so-called lockdown. As is well known, between March and June 2020, most European countries (and others) saw the closure of shops, bars, restaurants, gyms, etc., with the population urged not to leave their homes except for proven needs. Although lockdown is a very efficient measure for containing the virus, it cannot be applied for long periods of time, above all because of the impact it has on the economy and on the society of the countries that implement it.

For many countries, therefore, after a period of generalised lockdown, a phase has begun in which the population has been prepared to 'live' with the virus, both in an 'active' manner through the constant use of personal protection equipment (masks, gloves, etc.) and through the adoption of different behaviour and habits focused on prevention, keeping awareness high among people that the virus is circulating.

In this situation of coexistence with the virus, the accurate tracing of contacts among positive individuals is proving to be fundamental in order to prevent the spread of possible outbreaks of contagion. To this end, various countries have implemented systems of contact tracing at a national level. In Italy, for example, tracing is based on an application that is able to detect contacts using Bluetooth or similar technologies. A disadvantage of this technology is the low accuracy of the collected data, both due to the fact that the estimate of the distance between two devices is exposed to considerable error and to the fact that the data themselves might contain incomplete or missing information.

In general, the need has therefore been felt for a tracing process that allows accurate tracing of which people have come into contact with a reference person, particularly within defined spaces. Contact tracing is in actual fact a not so recent practice and several application methodologies have been explored in recent years, which still have considerable limitations.

For example, the publication "WiFiTrace: Network-Based Contact Tracing for Infectious Diseases Using Passive WiFi Sensing" by Trivedi, Zakaria, Balan and Shenoy (Trivedi et al.) of 26 May 2020 discloses a contact tracing system for containing the spread of infectious diseases by exploiting a set of detection points (access points) provided within a defined space.

The approach of this publication is based on WiFi Sensing technology without customer involvement and provides for a series of access points capable of detecting the passage of electronic devices, by WiFi sensing, thus constituting a WiFi network. When a user moves from one position to another, their mobile device (a mobile phone, tablet, etc.) associates via WiFi with an access point. Each access point can record the identification of the device and the moments of association and of disassociation and given that the position of each access point is known, it is possible to reconstruct the path of said device.

The technology presented in the publications of Trivedi et al. has, by the authors' own admission, limitations due to the lack of a precise association between the device that is detected and its real user. In fact, cases of users who use several mobile devices (two mobile phones, tablets, laptops, etc.) are not uncommon. The immediate consequence is that the count of detected devices is always higher than that of effective users and not all devices have associated information on their possession.

At the same time, not all users connect their device to the WiFi network and therefore cannot be detected by access points. Non-associated devices cause a series of clear consequences on the accuracy of the tracking method. It should also be considered that at times mobile devices can be shared between several users.

The limits of such an approach based on WiFi sensing technology are therefore evident, which approach does not allow a unique association between user and device to be defined, and which is based, for tracing, simply on a mapping of the information (hash) associated with the devices detected.

The publication "COVID-19 Contact Tracing: Challenges and Future Directions" by Chowdhury Mohammad Jabed Morshed et al. of 9 November 2020 analyses the most recent approaches for contact tracing, in particular with regard to the COVID-19 pandemic, highlighting the limitations associated with the use of specific applications for mobile devices and of approaches based on Wi-Fi detection.

From what is disclosed above, the limitations of currently known contact tracing systems, particularly those based on WiFi detection, are evident. To this end, the need for a method of unique association between the user and the medium that has to be detected in order to implement the tracing methods becomes even more apparent.

## Summary of the invention

The object of the present invention is therefore to provide a method that allows the tracing of contacts that have occurred among a group of people within a circumscribed space that can overcome the limitations of known technologies.

More particularly, an object of the present invention consists of providing a method for creating a list of users who have come into contact with a reference user in a given time interval.

A particular object of the present invention is to provide a method for tracing contacts that have occurred in spaces that have controlled access points and that can be applied in various contexts such as ski resorts, amusement parks, shopping malls, and so on.

These and other objects are achieved by a method for tracing contacts having the features listed in the appended independent claim 1.

Advantageous embodiments of the invention are disclosed by the dependent claims.

Substantially, the present invention relates to a method for tracing contacts among a group of users within a space in which a plurality of detection stations are provided, wherein each user of the group of users is assigned a tracing means suitable for being detected by the detection stations comprising the steps consisting of

- recording, by means of detection of said tracing means, all the passages performed by each user at each station of the plurality of stations, each of the passages having associated a time stamp relating to the instant of detection and the identifier of the tracing means;
- selecting a reference user from among the group of users, identifying for each station all the passages performed by said tracing means assigned to said reference user;
- associating each user of the group of users with a unique identification code and creating a database table in which the data and contact details of each user, together with the associated unique identification code, are recorded;
- associating said unique identification code with the tracing means assigned to each user;
- for each station, having established a probable contact interval, which is a time interval within which two users who have passed through the same station risk having been in contact, among all the passages determining possible passages of users who have come into contact with the reference user and after having determined these passages, through the relationship linking the passages, the tracing means and the unique identification codes, identifying the identity of the users who have come into contact with the reference user.

## Brief description of the drawings

Further features of the invention will be made clearer by the following detailed description, referring to purely illustrative, and therefore non-limiting, embodiments illustrated in the accompanying drawings, in which:

Figure 1 is an exemplary schematic illustration of the relationships that exist between a group of users and the detection stations provided in a space in which the tracing method according to the present invention is applied;
Figure 2 is a schematic diagram of the relationships between some of the tables used for contact tracing;
Figure 3 is an exemplary representation of a table functioning as a register of passages;
Figure 4 is a diagram showing the main steps of a process that implements the method according to the present invention to trace the contacts occurring within a ski facility; and
Figures 5 to 8 are flowcharts showing in greater detail the operations performed within the steps illustrated in Figure 4.

## Detailed description of the invention

A preferred embodiment of a method for tracing contacts occurring within a group of users will now be described in detail. The application is particularly advantageous when it is necessary to trace the contacts had by a person within spaces which, although large, are circumscribed by suitably arranged detection points.

A particularly advantageous application of the method according to the invention, which will be discussed in greater detail here below, allows tracing of contacts between users of ski resorts, occurring for example in proximity of ski lifts, exploiting data from ticketing systems and recording the passages of ski pass cards. This application however is absolutely not limiting, as the fields of application of the invention are many and varied.

The method according to the present invention allows definition of if and when contacts have occurred within a group of $n$ users U, with U = $\{U_1, U_2,..., U_n\}$, in particular if a reference user $U_{ref}$ has had contacts with any other user $U_i$ of the group U within a space in which a plurality $m$ of detection stations S are provided, where S = $\{S_1, S_2,..., S_m\}$.

The detection stations S are distributed in a circumscribed space, potentially without size limits, and are arranged in proximity of places in which concentrations of people could be created, such as a ski lift in a ski resort, a toilet inside a trade fair or shopping centre, an entrance turnstile to a hall, a tennis court inside a sports centre, an attraction in an amusement park, etc. The detection stations have the purpose of keeping both spatial and temporal track of the movements of individual users within the circumscribed space.

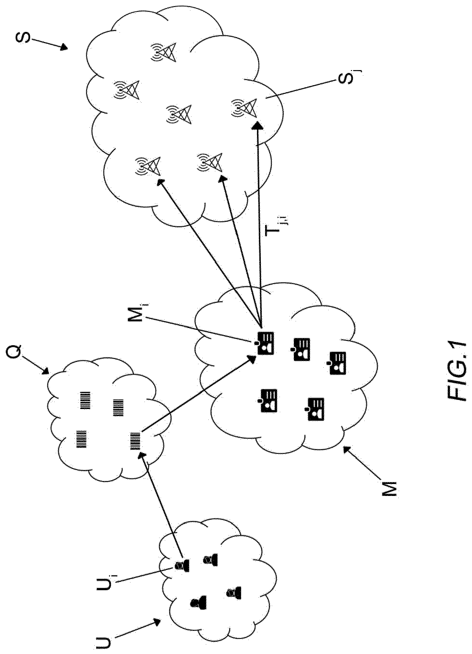

In order to make the contact tracing effective, the recording is provided of the personal data of the users and assigning to each user tracing means able to be read by the detection stations. Figure 1 is a simple illustrative schematic illustration of the relationships that exist between the sets that define the group of users U of a system and the plurality of detection stations S provided therein, through the use of various tracing means M.

The group of users U is not a rigid set and can take on different configurations according to requirements. It may in fact comprise only the users, or part thereof, who have frequented a ski resort on a given day, or it may be extended to new users who have frequented the ski resort on the following day, during the current week or month, and so on.

For the purpose of tracking and tracing the contacts that have taken place, a first step of the method according to the present invention therefore consists in assigning to each user $U_i$ of the user group U a tracing means $M_i$ that is designed to be detected by the detection stations S. Without any limitation, the tracing means may be any means suitable to be read by the stations S, whether it is a physical medium, such as a magnetic card issued by a ticket office, or in digital format or readable by software stored on electronic devices, such as a barcode, a QRcode or the like. Obviously, the stations S have the means such as to allow the reading of the tracking media, whether in physical format (magnetic cards, RFID, etc.) or in software-readable format, and may also provide for a combination of simultaneous reading systems.

As already highlighted in the description of the technological background of the invention, the situation often arises where the same tracing means, in particular if provided in physical media, is used several times by different users. This occurs for example also in some ski resorts, where the ski pass has a limited duration (a day, a weekend, a week, etc.) and is "loaded" on a magnetic card assigned to a user, who then gives it back at the end of the validity period. The returned card is then reassigned to a different user.

In order to make a tracking system $M_i$ assigned to a user $U_i$ an identification system, i.e. able to uniquely identify the user who has transited in front of the detection station, in an innovative way compared to known tracing methods a unique $Q_i$ identification code is associated with each user $U_i$. This operation can be carried out at the first "entrance" of a user, for example the first time the user accesses the ski resort, or in general the first time they use a service. Advantageously, this operation is completed by the creation and by the updating of a special table UR, called for example "User Identification Register" (or simply "User Data" in the drawings), in which for each user $U_i$ information is recorded useful for their tracking, including name and surname, address, telephone number, etc., as well as the unique identification code $Q_i$. The set of all unique identifiers assigned defines a set Q.

Once it has been established that each user $U_i$ in the group U has a unique identifier $Q_i$ which is associated on each occasion with a tracking medium $M_i$, the detection of said tracking medium $M_i$ makes it possible to identify unambiguously and without any possibility of confusion to which user $U_i$ the detected passage belongs, even if the tracking medium has been used previously by other users. To this end, it is advantageous to create a table AR, defined for example as "Assignment Register", in which each assignment of each tracing means $M_i$ is recorded, entering the date, time and the unique identification code $Q_i$ of the user $U_i$ to whom it has been assigned. The table AR allows, for example, given a date, a time and a card number, the unique identification code of the user holding the card at that time to be obtained.

The assignment register AR, as already pointed out, is particularly useful when the tracing means can be reassigned to different users, as in the example of reusable ski pass cards, or when the indication of the tracing means $M_i$ could be associated, at different times, to several identification codes and this does not allow the user to be identified uniquely. In the same way, the table could contain several times a single user $U_i$ in association with different tracing means used on different days. For this reason, in order to determine a single card possession or possession interval, it is necessary to define the tuple of values (tracking medium $M_i$, unique code $Q_i$, date and time of assignment).

An assignment register AR is also essential should the tracking medium $M_i$ be a tracking medium which is always and only used by a specific user, for example a rechargeable card which remains in the possession of the user $U_i$ and which cannot be reassigned. It may happen that, in the case of a rechargeable card, it is lost, or that a software-readable code is corrupted or can no longer retrieve the information. In these cases, a new tracing means has to be reassigned to the user.

As already anticipated, the tracing method according to the present invention is based on the provision of a plurality of detection stations S that enable the detection of passages made by the users through the tracing means in their possession. In this disclosure, the detection of a tracing means $M_i$ at a station Sj defines, together with the time of detection, a use or, with a preferred terminology, a passage $t_{j,i}$.

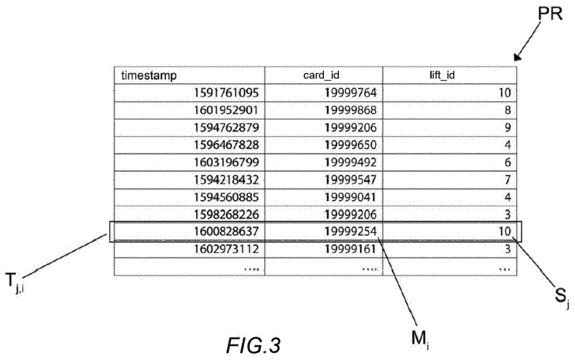

A specific table $PR_j$ is then created for each station Sj in which all passages $t_{j,i}$ of each tracking medium $M_i$ are automatically recorded. Conveniently, all the passages that occurred in all the stations S are recorded in a larger table PR, defined "Passage Register" or "Ticket Usages", which lists the passages $t_{j,i}$ detected in the whole application space of each tracing means $M_i$, each passage comprising information about the place of use (the detection station $S_j$) and a time stamp related to the detected instant, preferably in UNIX timestamp format. Figure 3 shows an example embodiment of a table defining a register of passages.

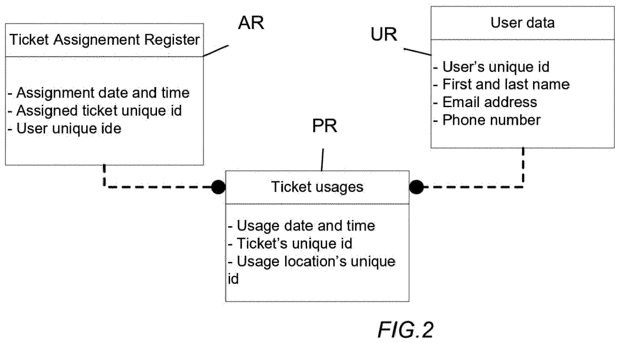

Figure 2 schematically shows a data model based on the relationship between the tables and registers defined above for the purpose of implementing the contact tracing method according to the present invention, which allows identification of contacts made by a specific reference user $U_{ref}$ in respect of other users of the group U.

For the purposes of the present invention, it is necessary to introduce the definition of *probable contact interval* $\Delta t$, i.e. the time interval within which two users who have passed through the same station Sj risk having been in contact. Contact, in this context, takes on more of a temporal meaning than a physical one, meaning therefore a window of time in which two parties have passed through substantially the same point. For example, two temporally successive passages at a detection station Sj provided at a ski lift indicate that, most likely, two users have been in physical proximity in the entrance queue to the lift and that therefore a 'contact' has occurred, even if not necessarily physical.

The probable contact interval $\Delta t$ therefore defines a window of time (which can be selected according to various parameters) within which it is probable that several users have shared a space and is a parameter that can generally vary according to the specific detection station also within the same scope of application. For example, a detection station Sj located at a chairlift within a ski resort, can have a probable contact interval $\Delta t_j$ that is lower than that of another station $S_{jj}$ in which access is systematically slower. In general, each station $S_j$ is placed at detection points that can have different transit conditions and that determine a specific probable contact interval $\Delta t_j$.

Once the user $U_{ref}$ whose contacts are to be traced has been defined, identification is carried out of the tracking medium (media) $M_{ref}$ assigned thereto by means of appropriate queries of the assignment register AR. The identification can be carried out on the whole register, or on a limited portion defined by *an interval of interest* (or risk interval) $\Delta r$, i.e. the interval of time within which there is interest in tracing the contacts made by the reference user $U_{ref}$. Specifically, once the unique identifier $Q_{ref}$ has been retrieved for the reference user $U_{ref}$, the tracing means $M_{ref} = \{M_{ref,1}, M_{ref,2}, ..., M_{ref,k}\}$ assigned to the user in the interval of interest $\Delta r$ are identified and the instants of beginning and of end of possession (or use) are found, thus obtaining a new table $IR_{ref}$ called "Intervals of possession". In general, the operation is carried out for all tracking media used by all users, generating a global possession intervals table IR.

The identifiers of the tracking media $M_{ref}$ associated with the reference user $U_{ref}$ and the relative intervals of use are used to perform a query of the passage register PR. It should be recalled that in this table all detected passages for all tracking media at all tracking stations are entered and each record contains at least these three pieces of information (attributes): the identifier of the detected tracking medium, the detection station at which the transit is carried out and the instant of detection. Fig. 3 shows an example of such a table PR, in which each instant of detection is entered in UNIX timestamp format and corresponds to the day, month, year, hour, minute and second.

Without going into details that are evident to persons skilled in the art, the table PR is filtered considering each of the tracing means $M_{ref}$ associated with the reference user (via the identification code or the like) and the relative possession interval (via the attribute relating to the instant of detection). This query identifies the passes $t_{j,ref}$ made by the reference user at each station $S_j$, if any. A different approach, which nevertheless leads to the same result, is based on the query of the tables of the passages $PR_j$ specific to each single station, aimed at verifying possible passages of the cards $M_{ref}$ in the possession interval.

Having established the passages $t_{j,ref}$, i.e. all the instants in which a reference user $U_{ref}$ has passed through one $(S_j)$ of the plurality of stations S, it is then possible to immediately trace the passages of users that may have come into contact with this reference user. In particular, a passage in front of a detection station $S_j$ is a passage of a user who has come into contact ($t_{j,contact}$) with the reference user $U_{ref}$ if the following condition is in place:

$$t_{j,ref} - \Delta t_j < t_{j,contact} < t_{j,ref} + \Delta t_j$$

where $\Delta t_j$ represents the aforementioned interval of probable contact valid for the j-th station.

After having determined the passages of users who have come into contact tj,contact, the identification of these users is immediate, remembering that for each passage $t_{j,i}$ the detected tracking medium $M_i$ is recorded, which in turn is associated with a unique user identification code $Q_i$. A list $CL_j$ is then obtained, for each station $S_j$, of the users that have come into contact with the reference user. The operation is repeated for all stations S and the tables can be combined in a global register CL containing all the passages of users who came into contact, the time and place (station) of detection.

In certain situations, it may be foreseen for various reasons that some users do not have to be tracked even though they are part of the set of those who came into contact with the reference user. For this purpose, an NCL list containing the unique identifiers of the users not to be contacted can be provided, which can be compiled for example when assigning tracing means or at the same time as updating of the assignment register AR. If there are unique identifiers in the list of contacts CL that are also present in the table NCL, these will be removed from the list of users to be contacted. Naturally, such a table NCL is subject to change, as the list of users not to be contacted may vary from time to time. It can be emptied at the end of a period of time and repopulated at the beginning of the next period or updated by inserting new users not to be contacted and removing items initially present.

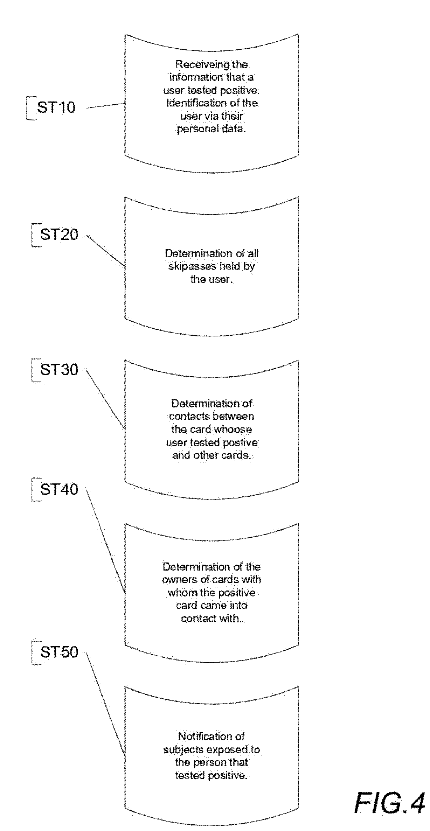

The method described above makes it possible in general to trace the contacts that a reference user has had for a period of time within a delimited space. A particularly advantageous embodiment of the invention is represented by the application of the method with the aim of tracing the contacts that a user has had within a ski resort or an amusement park in which there are multiple attractions, shops and food outlets. An even more advantageous, but by no means restrictive, application enables tracing of the contacts of a user found to be positive for particularly contagious diseases, as in particular the aforementioned COVID-19. An example application of this method within an MS management system of a ski resort is then described in greater detail, with particular reference to the diagrams illustrated in Figures 4 to 8. The application in an amusement park is to be considered substantially similar.

Figure 4 is a diagram summarising the flow of the process for tracing the contacts made by a user of a ski resort. The process starts (phase ST10) with the receipt by the system SM of a notification that a user $U_{ref}$ who has recently attended the facility has tested positive. The user is immediately identified thanks to the information present in the users register UR present in the facility management system.

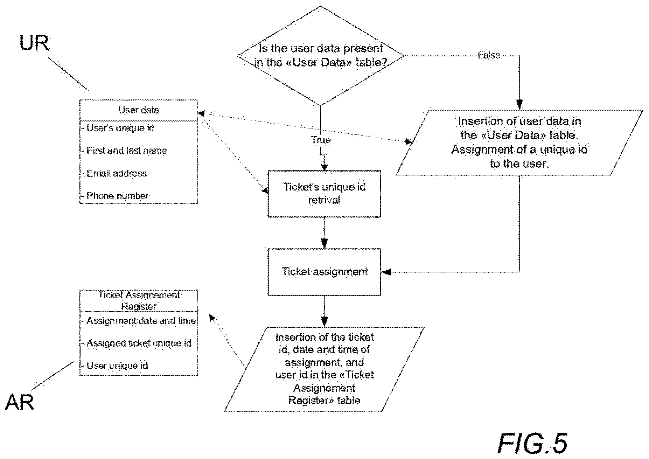

The table UR, as already described in the general discussion of the tracing method, collects information on all users who have had access to the facility and is constantly updated during normal administration of the ski resort. Figure 5 is a flowchart showing the process of acquisition of user information, in particular carried out in combination with a ticketing system that issues ski passes (or entry tickets to the amusement park) in the form of rechargeable magnetic cards.

Although an application will be described in which the cards assigned to a user are returned at the end of use and reassigned to a different user, the same methodology can also be applied to personal cards, which do not have to be returned but only "charged", without departing from scope of this discussion.

Referring to Fig. 5, when a user goes to the ticket office to purchase a ski pass, it is checked whether their data are already present in the archives. In the case wherein a user $U_i$ purchases a ski pass for the first time at the ticket office of the facility, they will be registered by acquiring their contact details and assigning them a unique identification code $Q_i$, updating the users register UR of the facility. In this phase it is possible to provide for the compilation of an NCL list of users not to be contacted.

Once the user's unique code has been established, which is already available in the case of a user already present in the register, a card is assigned which serves as a tracing means $M_i$. The date of assignment of the ski pass cards and the user code to which the card has been assigned at each purchase are also recorded, updating the information in the assignment register AR each time. This last step is essential given that the cards are returned to the ticket office after use and reassigned to different users.

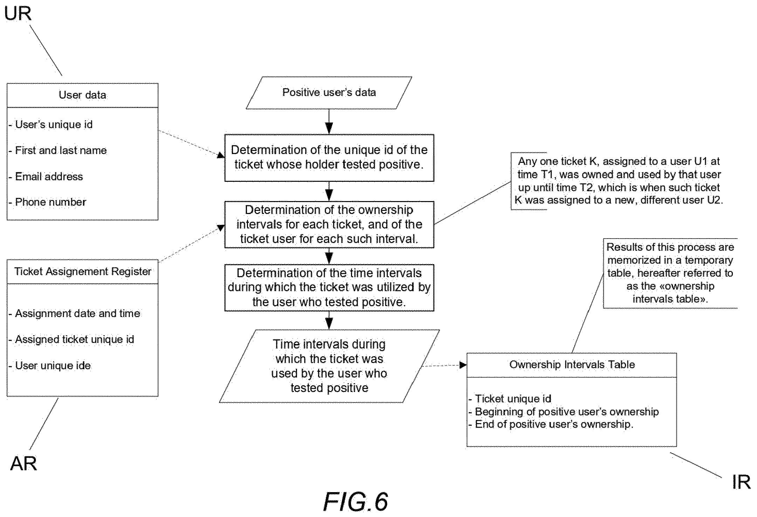

As already highlighted, the table AR makes it possible, given a date, a time and a number of ski pass card, to obtain the unique identifier of the user in possession of the card at that time. Referring again to Fig. 4, this operation is carried out in the ST20 phase, to identify the cards used by the user $U_{ref}$ who has tested positive and the time intervals in which they have been in possession thereof. The process of determining the intervals of possession is schematised by the flowchart illustrated in Fig. 6.

For example, the elements of the assignments register AR having the same tracing means (e.g. the code of the ski pass card most recently used by the positive user) are considered and they are ordered according to the time of assignment. If the first assignment of the card took place at instant $t_1$ for user $U_1$, the second assignment took place at instant $t_2$ for user $U_2$ and so on, it follows that a generic user $U_i$ has held the card from ti+i to $t_i$. In the case wherein no registration subsequent to $t_i$ is registered, the instant of termination of possession of the card is considered, limited to the object of this application, as the moment of execution of the tracing method.

The process described is repeated for each card assigned in a period of time, starting from the date of execution of the method, at least equal to the interval of interest $\Delta r$ (or in this case risk interval) and which is in any case such as not to require excessive calculation power.

At the end of this step we obtain the table IR (Intervals of Possession), in which will be recorded, for each ski pass card, all the users who have used it, together with the interval of time during which they used it. This table can then be properly filtered, leaving only the data relating to the possession of the cards by the positive user.

Having obtained the numbers of the cards assigned to the positive user and the relative intervals of possession, identification is carried out (ST30 in Fig. 4) of which other cards have transited in proximity to the cards assigned to the positive user during said intervals of possession. As already mentioned, two users who use their own card at the same station $S_j$ and within a defined interval of probable contact $\Delta_{tj}$ can be defined as a contact, being probable both the physical proximity in the queue leading, for example, to the ski lift, and the proximity during the i, whether using a gondola lift or a chairlift.

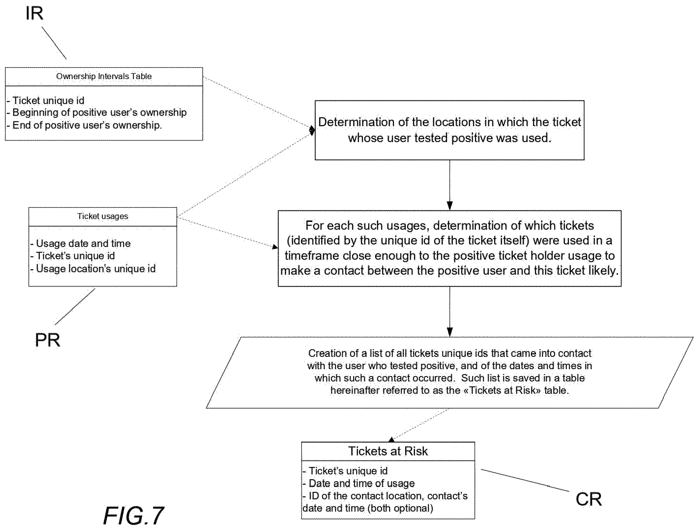

The procedure initially is to retrieve, from the table PR in which all the passages made by the cards in the detection stations are recorded, according to the methodology described in the general application of the method and illustrated in the diagram of Fig. 7, in combination with the tables $IR_{ref}$ (intervals of possession), the passages $t_{j,ref}$ made by the cards in the name of the positive user at each station, in the time interval in which they were the owner thereof.

Performing this procedure for all the other cards used within the risk interval, combining the register of passages PR with the possession intervals IR of the individual cards, all the passages $t_{j,i}$ made by the i-th card at each station Sj are then determined and it is established whether it had contacts, in that particular station, with the positive user $U_{ref}$. It should be remembered that a passage $t_{j,i}$ is a passage of a user who has come into contact $t_{j,contact}$ with the positive user if the following condition occurs:

$$t_{j,ref} - \Delta t_j < t_{j,contact} < t_{j,ref} + \Delta t_j$$

The result of this operation, repeated for all stations S, is the creation of a temporary table CR (which can be defined, for example, "tickets at risk" or "cards in close proximity" in the drawings) in which all the cards with which the positive user has come into contact are listed, with the place and time at which the contact took place.

Having identified the cards that were detected in proximity of the positive user (contact), with the times and places of detection, it is necessary to determine the owners of the cards at the time of contact (ST40, Fig. 4).

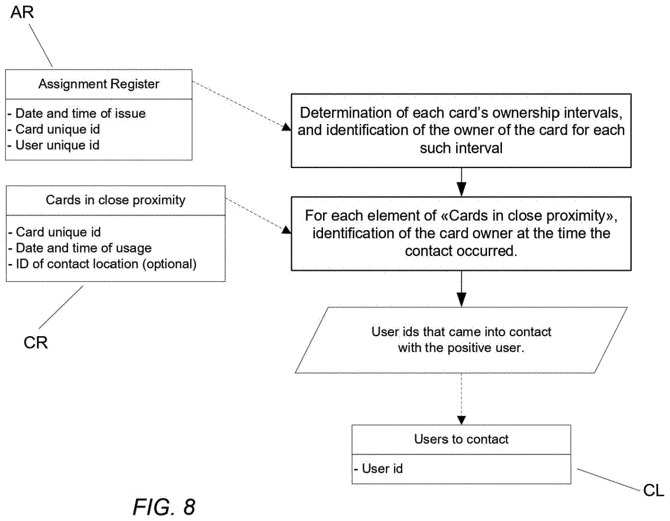

As illustrated in the flowchart of Fig. 8, starting from the register of the assignments AR and from the table of the cards in proximity CR, it is possible to trace the user codes of the tracked cards, from which it is possible to trace the identity of the users, creating a suitable list of the users to contact CL.

The final step in the process consists of notifying (ST50) the users involved by the fact that they have been in contact with a positive subject, using the data recorded in the user register UR.

The method for the tracing of contacts, described both in terms of overall functioning and with reference to a preferred embodiment, generally allows for the tracing of contacts occurring among users of controlled access facilities and structures, including underground lines, buses, trains, chairlifts, gondola lifts, amusement parks and other similar facilities, guaranteeing a combination of accuracy and ease of use by the end user that is superior to that of currently implemented systems.

The time between the use, in the same place, of two tracing means, associated for example with entrance tickets, can be found to be an indicator of contact occurring between two users, with the interval of likely contact that varies according to the specific application. For example, two public transport users who board a bus a few minutes apart one from the other can be considered to have been in contact, it being likely that they have been in proximity to each other in the queue to board the bus or within the vehicle itself.

It is possible to implement the method within any area controlled by an access control system, by placing appropriate detection stations in the points wherein higher concentrations of users could be created, for example toilets inside a trade fair hall. Upon notification of a positive user, it will be easy to check the people who have used the same toilet as the positive user before the scheduled disinfection.

The tracing method can be used in a variety of applications, even in facilities that do not have a ticketing system or that use access systems not necessarily based on tickets or magnetic cards. For example, two users who use contactless payment systems to enter the underground may have been in contact if their entrances occurred within a limited time interval.

The method can also be applied to very large spaces, such as a network of roads with toll access points. Detection stations can be provided at restaurant facilities along the road, tracking the date and time of entry and exit of each user, thus being able to identify which other people have stayed at the rest stop during the same time period.

The method of contact tracing according to the present invention, exploiting the association of each user with a unique identification code which is associated on each occasion with a specific means intended to be detected by the detection stations, thus allows overcoming of the limits of the known technologies and proposes itself as a particularly valid and precise tracing tool for active control of contacts.

Naturally, the invention is not limited to the particular embodiments previously described and illustrated in the accompanying drawings, but numerous detailed changes may be made thereto within the reach of the person skilled in the art, without thereby departing from the scope of the invention as defined by the appended claims.

**Claims**

1. Method for tracing the contacts that have occurred among a group of users (U) within a space in which a plurality of detection stations (S) are set up, wherein each user ($U_i$) of the group of users (U) is assigned a tracing means ($M_i$) apt to be detected by the detection stations (S), comprising the steps consisting of

   - recording, by detecting said tracing means ($M_i$), all the passages ($t_{j,i}$) carried out by each user ($U_i$) at each station (Sj) of the plurality of stations (S), each of the passages ($t_{j,i}$) having associated a time stamp related to the detection time and the identifier of the tracing means ($M_i$);
   - once a reference user ($U_{ref}$) has been selected from among the group of users (U), identifying for each station (Sj) all the passages ($t_{j,ref}$) made by said tracing means ($M_{ref}$) assigned to said reference user ($U_{ref}$);
- for each station (Sj), having established a probable contact interval ($\Delta t_j$), which is an interval of time within which two users who have transited through the same station (Sj) risk having been in contact, among all the passages (tj,i), determining possible passages ($t_{j,contact}$) of users who came into contact with the reference user ($U_{ref}$) such that the following condition has occurred:

$$t_{j,ref} - \Delta t_j < t_{j,contact} < t_{j,ref} + \Delta t_j$$

**characterised in that** it comprises the steps consisting of:

- associating with each user ($U_i$) of the group of users (U) a unique identification code ($Q_i$) and creating a user identification register (UR) in which the data and contact details of each user ($U_i$), together with the associated unique identification code ($Q_i$) are recorded;
- associating said unique identification code ($Q_i$) with the tracing means ($M_i$) assigned to each user ($U_i$); and
- from the passages ($t_{j,contact}$) determined identifying, through the relation linking the passages ($t_{j,i}$), the tracing means ($M_i$) and the unique identification codes ($Q_i$), the identity of the users who came into contact with the reference user ($U_{ref}$).

2. Method according to claim 1, wherein the identities of the users who came into contact with the reference user are collected in a list of users to be contacted (CL).

3. Method according to claim 2, further comprising the steps consisting of:

- compiling a list of users not to be contacted (NCL) including, for each user, the respective unique identifier;
- removing from said list of users to be contacted (CL) the users also present in the list of users not to be contacted (NCL).

4. Method according to any one of the preceding claims, wherein the identification of the passages ($t_{j,ref}$) made by said tracing means ($M_{ref}$) assigned to the reference user ($U_{ref}$) is limited in time to an interval of interest ($\Delta r$).

5. Method according to any one of the preceding claims, wherein said tracing means (M) is represented by physical media, such as magnetic cards, RFID-based systems or the like, software-readable media, such as barcodes, QR codes or the like, or by a combination of physical media and software-readable media.

6. Method according to any one of the preceding claims, wherein each of said plurality of detection stations (S) has instruments able to detect said tracing means both in physical and software-readable format.

7. Method according to any one of the preceding claims, wherein tracing means ($M_i$) is represented by a card used as ski pass in ski resorts or as an entry ticket to an amusement park.

8. Method for contact tracing according to claim 7 carried out in combination with a system of management (MS) of a ski resort or of an amusement park, comprising the steps consisting of:

- receiving the report (ST10) of the need to identify the contacts that a reference user ($U_{ref}$) had with other users within a group of users (U);
- determining (ST20) the card ($M_{ref}$) owned by the reference user ($U_{ref}$);
- determining (ST30) the contacts occurring between the card owned by the reference user ($M_{ref}$) and other cards used by the group of users (U);
- determining (ST40) the holders of the cards with which the reference user has had contacts;
- contacting (ST50) the users identified in the previous step (ST40).

9. Method according to claim 8, wherein said reference user is a COVID-19 positive user.

FIG.1

AR

Ticket Assignement Register

- Assignment date and time
- Assigned ticket unique id
- User unique ide

UR

User data

- User's unique id
- First and last name
- Email address
- Phone number

PR

Ticket usages

- Usage date and time
- Ticket's unique id
- Usage location's unique id

FIG.2

EP 4 006 911 A1

| timestamp | card_id | lift_id |
|---|---|---|
| 1591761095 | 19999764 | 10 |
| 1601952901 | 19999868 | 8 |
| 1594762879 | 19999206 | 9 |
| 1596467828 | 19999650 | 4 |
| 1603196799 | 19999492 | 6 |
| 1594218432 | 19999547 | 7 |
| 1594560885 | 19999041 | 4 |
| 1598268226 | 19999206 | 3 |
| 1600828637 | 19999254 | 10 |
| 1602973112 | 19999161 | 3 |
| .... | .... | ... |

$PR$

$T_{j,i}$

$M_i$

$S_j$

*FIG.3*

ST10 — Receiveing the information that a user tested positive. Identification of the user via their personal data.

ST20 — Determination of all skipasses held by the user.

ST30 — Determination of contacts between the card whoose user tested postive and other cards.

ST40 — Determination of the owners of cards with whom the positive card came into contact with.

ST50 — Notification of subjects exposed to the person that tested positive.

*FIG.4*

FIG.5

**Is the user data present in the «User Data» table?**

False → Insertion of user data in the «User Data» table. Assignment of a unique id to the user.

True → Ticket's unique id retrival → Ticket assignment → Insertion of the ticket id, date and time of assignment, and user id in the «Ticket Assignement Register» table

**UR**
User data
- User's unique id
- First and last name
- Email address
- Phone number

**AR**
Ticket Assignement Register
- Assignment date and time
- Assigned ticket unique id
- User unique id

UR

FIG.6

EP 4 006 911 A1

**User data**

- User's unique id
- First and last name
- Email address
- Phone number

**Ticket Assignement Register**

- Assignment date and time
- Assigned ticket unique id
- User unique ide

AR

Positive user's data

Determination of the unique id of the ticket whose holder tested positive.

Determination of the ownership intervals for each ticket, and of the ticket user for each such interval.

Determination of the time intervals during which the ticket was utilized by the user who tested positive.

Time intervals during which the ticket was used by the user who tested positive

Any one ticket K, assigned to a user U1 at time T1, was owned and used by that user up until time T2, which is when such ticket K was assigned to a new, different user U2.

Results of this process are memorized in a temporary table, hereafter referred to as the «ownership intervals table».

**Ownership Intervals Table**

- Ticket unique id
- Beginning of positive user's ownership
- End of positive user's ownership.

IR

IR

| Ownership Intervals Table |
| --- |
| - Ticket unique id<br>- Beginning of positive user's ownership<br>- End of positive user's ownership. |

Determination of the locations in which the ticket whose user tested positive was used.

| Ticket usages |
| --- |
| - Usage date and time<br>- Ticket's unique id<br>- Usage location's unique id |

For each such usages, determination of which tickets (identified by the unique id of the ticket itself) were used in a timeframe close enough to the positive ticket holder usage to make a contact between the positive user and this ticket likely.

PR

Creation of a list of all tickets unique ids that came into contact with the user who tested positive, and of the dates and times in which such a contact occurred. Such list is saved in a table hereinafter referred to as the «Tickets at Risk» table.

| Tickets at Risk |
| --- |
| - Ticket's unique id<br>- Date and time of usage<br>- ID of the contact location, contact's date and time (both optional) |

CR

*FIG.7*

*FIG. 8*

EP 4 006 911 A1

Europäisches
Patentamt

European
Patent Office

Office européen
des brevets

## EUROPEAN SEARCH REPORT

Application Number

**EP 21 21 1049**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TRIVEDI AMEE ET AL: "WiFiTrace: Network-based Contact Tracing for Infectious Diseases Using Passive WiFi Sensing", , 26 May 2020 (2020-05-26), pages 1-23, XP055828894, Retrieved from the Internet: URL:https://arxiv.org/pdf/2005.12045v2.pdf [retrieved on 2021-07-29] * The whole document, in particular: Pages 1, 7 - 11, 21; Figures 1 - 4. * ----- | 1-9 | INV. G16H10/60 |
| A | CHOWDHURY MOHAMMAD JABED MORSHED ET AL: "COVID-19 Contact Tracing: Challenges and Future Directions", IEEE ACCESS, IEEE, USA, vol. 8, 9 November 2020 (2020-11-09), pages 225703-225729, XP011827346, DOI: 10.1109/ACCESS.2020.3036718 * The whole document, in particular: Abstract; Pages 225705, 225706. * ----- | 1-9 | TECHNICAL FIELDS SEARCHED (IPC) G16H |

The present search report has been drawn up for all claims

| Place of search | Date of completion of the search | Examiner |
|---|---|---|
| Munich | 30 March 2022 | Nagele, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)